# EUROPEAN PATENT APPLICATION

(11) **EP 1 607 073 A2**
(43) Date of publication of application: **21.12.2005**
(21) Application number: 05018153.6
(22) Date of filing: 07.03.2000
(51) Int. Cl.: A61F 5/08, A61D 9/00, A61F 13/12

(54) **Nasal support device for domestic mammals**

(30) Priority: 08.03.1999 US 264464; 23.08.1999 US 379425; 15.11.1999 US 165578 P
(62) Divisional of application: 00917779.1
(71) Applicant: Winease LLC, Eagan, MN 55123-2434 (US)
(72) Inventor: Blach, Edward, L., Roswell, New Mexico 88201 (US); Chiapetta, James, R., Eagan, Minnesota 55123-2434 (US); Cohen, Daniel, E., Eden Prairie, Minnesota 55347 (US)
(74) Representative: Humphreys, Ceris Anne

(57) **Abstract**

The present disclosure provides a device for facilitating air flow in the nasal passage of a domestic animal. The nasal support devices (NSD) disclosed herein are useful for facilitating air flow during rest, physical exertion, respiratory ailment, etc. Components and methods to facilitate application of the support device to the nose of an animal are also disclosed.

## Description

### Field of the Invention

The present invention is directed to facilitating air flow through the nasal passages of a domestic animal. Specifically, the invention provides devices and methods for supporting the soft tissue structures of the nasal passages of a domestic animal.

### Background of the Invention

Portions of the following discussion of the nasal anatomy of domestic mammals are excerpted from R. Nickel et al., *The Viscera of Domestic Animals,* (2nd revised ed.), Springer-Verlag, New York, Hiedelberg, Berlin (1979), pp. 211-221. This is an excellent text on the comparative visceral anatomy of domestic mammals. As used herein, the terms "mammal" and "animal" are used synonymously and refer to non-human mammals.

The nasal anatomy of domestic animals is considerably different than that of a human. Unlike the human nose that projects distinctly from the face, in domestic animals, the nose is incorporated into the face and forms the large dorsal and lateral areas rostral to the eyes. The nostrils in the apex of the nose are the entry to the respiratory system of domestic mammals. Once passing through the nostrils, inspired air moves into the nasal cavities and continues through the nasopharynx, larynx, trachea and lungs.

At the apical entrance to the nose the nostrils are partitioned by the nasal septum to divide the nasal cavity into right and left halves. The caudal portion of the septum is bony, while rostrally the septum consists of cartilage which becomes progressively more flexible toward the apex.

The wall of the nose consists of skin externally and a middle supporting layer of bone caudally and cartilage rostrally. The nasal cavity is lined by a mucous membrane. The rostral bones forming the wall of the nose include the nasal, maxillary and incisive bones. The free borders of the nasal and incisive bone provide attachment for the cartilages which support the nostrils. The supporting bones and cartilages of the nose are associated with the nasal muscles that regulate the size of the nostrils.

The dorsal and ventral lateral nasal cartilages are formed by the widening of the rostral part of the nasal septum along its dorsal and ventral margins. In the horse, the ventral lateral nasal cartilage is small and may be absent. In many domestic animals, there is no lateral support for the soft tissue over the rostral nasal passage caudal to the nostril.

A further difference in the formation of the nasal cartilages of the horse is the presence of alar cartilages. The alar cartilages consist of a ventral cornu and a dorsal lamina and support the nostrils dorsally, medially and ventrally. The lamina of the alar cartilage and the medial accessory cartilage support the nasal diverticulum, a blind pouch in the dorsal aspect of the nostril.

The muscles of the nose and upper lip act to dilate the nostrils. This is particularly noticeable during labored breathing. In the horse, these muscles are well developed and can transform the normally semilunar nostrils to become circular.

The dorsal lateral area of the rostral nasal cavity that is caudal to the alar cartilages in the nostrils of the horse includes a region of unsupported soft tissue which can be drawn into the nasal cavity during inspiration of air into the nasal passages. The nasal diverticulum of the horse is a part of the soft tissue structures of the horse which can be drawn into the nasal cavity. When the soft tissue is drawn in, it can narrow the nasal cavity and reduce the area for the intake of air, thus reducing the air movement into the nasal passages and ultimately to the lungs where the oxygen is transferred in the pulmonary aveoli.

The physiological effects of reduced oxygen transfer at rest and during physical exertion are documented. Some experts have theorized that exercise induced pulmonary hemorrhage (EIPH) in performance horses is caused by asphyxia due to abnormal resistance of a closed or partially closed upper airway. The upper airway being defined as the region of the respiratory tract lying between the nostrils and the windpipe at the level of the first rib. Hence, the nasal passages are part of this region. Dr. Robert Cook, "EIPH or AIPE? A Tufts University Researcher suggests that bleeding is not caused by EIPH, but by asphyxia", The Equine Athlete, p. 22-23 (March /April 1997).

Devices for dilating the outer wall tissue of the nasal passages in humans have been described in, for example, U.S. Patent Nos. 5,653,224; 5,611,333; 5,533,503; 5,549,103; 5,546,929; 5,553,605; 5,476,091 and RE 35,408. Devices for supporting outer tissues of the nasal passages of animals must address the unique soft tissue and mechanical characteristics of an animal nose, particularly performance animals such as horses, camels and dogs. Support devices for the nasal passages of animals are known and described in, for example, U.S. Patent No. 5,913,873 and International Patent Publication WO 98/47451. The entire disclosure of the foregoing patent and patent application are incorporated herein by reference.

### Summary of the Invention

The present invention provides support devices and methods for using the support devices to support the unsupported nasal tissues of a domestic animal. The invention addresses providing support in view of the structural and physiological characteristics unique to the nose of a non-human animal, for example, a horse. In addition to providing features which enhance the function of a support device during use, the invention also provides components to facilitate the ease and accuracy of positioning a device on an animal's nose. The invention further provides features which permit reuse of a nasal support device after removing from an animal's nose and also addresses some of the needs which arise when using a support device during certain competitive events. It will be appreciated that some of the components or features of the herein disclosed devices may also be applicable for use with human nasal dilators.

Throughout the specification, guidance may be provided through lists of examples. In each instance, the recited list serves only as a representative group. It is not meant, however, that the list is exclusive.

In one embodiment the invention is a nasal support device ("NSD") for securing to the nose of the animal. In a typical embodiment, the NSD provides support to the right and left lateral vestibular walls of a domestic animal. The device includes a support layer and a right and left side piece which when secured to the nose of the animal are positioned to provide structural support to the right and left lateral vestibular walls. The side pieces of the device can meet at the midline region of the device. In some embodiments, when secured to the nose of a domestic animal, the midline region of an NSD of the invention straddles the left and right nasal bones of the animal.

The sides and the midline region of the NSD each have a rostral end, a caudal end and a rostral-poll dimension. In some embodiments, the rostral-poll dimension at the midline region can be equal to or greater than either of the rostral - poll of the side pieces. In addition, in some embodiments, the device is bilaterally symmetrical across both the longitudinal and transverse axes of the device.

In general, a support device according to the invention typically includes a surface layer, a support layer, and an engaging layer. The support devices can also include a carrier layer to facilitate handling and positioning of the device on an animal's nose. In some preferred embodiments, the support device is a dark color.

In alternative embodiments the invention provides facilitation to air flow in the nasal passages of an animal by supporting the caudal apex region of the vestibular walls of the animal. The devices are suitable for adult and young animals. In general, the structural aspects of an NSD of the invention can be configured and sized to fit the nose of, for example, a dog, cat, human, horse, camel, etc.

The disclosed support devices can be used on an animal that is running free in a pasture, or wearing saddlery, harnesses or other equipment that may be attached to the nose of the animal while performing physical activity.

The devices and methods of the invention are particularly advantageous for use in horses and are beneficial for use during athletic performance or for reducing the occurrence, severity or effect(s) of respiratory diseases in an adult or young animal.

In some embodiments, the NSD can be a reusable device. Methods for reusing an NSD are also disclosed.

### Brief Description of the Drawings

FIG. 1 is a top plan view of one embodiment of a nasal support device according to the invention;
FIG. 2 is a profile view of the bony anatomy of the rostral nasal cavity of the horse;
FIG. 3 is a bottom exploded view of the nasal support device of FIG. 1;
FIG. 4a is a top view of the nasal support device of FIG. 1 with the support layer illustrated in phantom;
FIG. 4b is a top view of one embodiment of lift members according to the invention.
FIG 5 is a top plan view of the nasal support device of FIG. 1 having a carrier layer;
FIG. 6 is a front view of a horse having the embodiment of a nasal support device of FIG. 1 secured to its nose;
FIG. 7 is a top plan view of the nasal support device of FIG. 1 having an alternative embodiment of a carrier layer;
FIG. 8 is a top plan view of an alternative embodiment of a nasal support device according to the invention;
FIG. 9 is a diagrammatic representation of an alternative embodiment of a nasal support device according to the invention; and
FIG. 10 is a diagrammatic representation of another alternative embodiment of a nasal support device according to the invention.
FIG. 11 is a diagrammatic representation of a configurational appearance for an NSD according to the invention having two axes bilateral symmetry;
FIG. 12 is a top side view of one appearance of an NSD having two axes bilateral symmetry;
FIG. 13 is bottom side view of the NSD of FIG. 12;
FIG. 14 is an alternative embodiment of a bottom side view of the NSD of FIG. 13;
FIG. 15 is an alternative embodiment of a bottom side view of the NSD of FIG. 13;
FIG. 16 is an alternative embodiment of a top side view of an NSD having two axes bilateral symmetry;
FIG. 17 is bottom side view of the NSD of FIG. 16;
FIG. 18 is an alternative embodiment of a bottom side view of the NSD of FIG. 16;
FIG. 19 is an alternative embodiment of a bottom side view of the NSD of FIG. 16;
FIG. 20 an alternative embodiment of a top side view of an NSD having two axes bilateral symmetry;
FIG. 21 is bottom side view of the NSD of FIG. 20;
FIG. 22 is an alternative embodiment of a bottom side view of the NSD of FIG. 20;
FIG. 23 is an alternative embodiment of a bottom side view of the NSD of FIG. 20;
FIG. 24 an alternative embodiment of a top side view of an NSD having two axes bilateral symmetry;
FIG. 25 is bottom side view of the NSD of FIG. 24;
FIG. 26 is an alternative embodiment of a bottom side view of the NSD of FIG. 24;
FIG. 27 is an alternative embodiment of a bottom side view of the NSD of FIG. 24;
FIG. 28 is an alternative embodiment of a bottom side view of the NSD of FIG. 24;
FIG. 29 an alternative embodiment of a top side view of an NSD having two axes bilateral symmetry;
FIG. 30 is bottom side view of the NSD of FIG. 29;
FIG. 31 is an alternative embodiment of a bottom side view of the NSD of FIG. 29;
FIG. 32 is an alternative embodiment of a bottom side view of the NSD of FIG 29;
FIG. 33 an alternative embodiment of a top side view of an NSD having two axes bilateral symmetry;
FIG. 34 is bottom side view of the NSD of FIG. 33;
FIG. 35 is an alternative embodiment of a bottom side view of the NSD of FIG. 33;
FIG. 36 is an alternative embodiment of a bottom side view of the NSD of FIG. 33;
FIG. 37 is an alternative embodiment of a bottom side view of the NSD of FIG. 33;
FIG. 38 is an alternative embodiment of an NSD according to the invention;
FIG. 39 is a rostral end view of the NSD of FIG. 38;
FIG. 40 illustrates an alternative embodiment of an NSD positioned over the lateral vestibular walls of a horse;
FIG. 41 is an alternative embodiment of an NSD according to the invention; and
FIG. 42 is a bottom plan view of one embodiment of a therapeutic device according to the invention.

### Detailed Description of the Invention

The invention is directed to devices and methods for supporting the soft tissue at the rostral aspect of the nasal cavity of domestic mammals. Specifically, the devices and methods disclosed provide support for the unsupported region of the "vestibule" in the rostral nasal cavity. While the components and features of the devices disclosed are particularly advantageous for animal devices, some of the components and features may also be advantageously used with human nasal dilators.

As used herein, domestic mammals include most non-human production and performance animals having a nose incorporated into the face, rather than projecting therefrom, that can benefit from a device according to the invention. Such mammals include dogs, cats, sheep, goats, cattle, horses, camels, llamas, etc. A device according to the invention can be particularly useful for members of the Equidae family including horses, donkeys, mules, zebras etc.

As used herein, "performance activities" or "work" includes activities such as pulling, driving, racing (flat, steeple, barrel, etc.), eventing, hunting, jumping, rodeoing, trail riding, endurance riding, etc. In general, the device can be used anytime it is desired to facilitate or enhance nasal air intake. In addition, to facilitating air flow, an NSD according to the invention can also be used to treat or prevent respiratory ailments in adult or young animals such as foals and calves. The devices and methods of the invention may be particularly beneficial in horses for reducing the severity or effect(s) of respiratory conditions such as laryngeal hemiplegia, chronic obstructive pulmonary disease (COPD) or exercise related pathologies such as myositis, dorsal displacement of the soft palate (DDSP), exercise induced pulmonary hemorrhage (EIPH) or "bleeding," etc.

As used herein, the term "rostral" refers to that aspect of the nose or anatomical structure closest to the apex of the nose. "Caudal" refers to that aspect of the nose closest to the poll or caudal aspect of the head relative to the apex. The "vestibule" refers to the rostral aspect of the nasal cavity that is defined by the alar cartilages rostrally, the incisive bone ventrally, the nasal bone dorsally, the caudal intersection of the incisive and nasal bones caudally, and the nasal septum medially. Thus, supported regions of the vestibule are supported by bone or cartilage.

The "unsupported" region of the vestibule is also referred to as the "lateral (free) wall" of the vestibule or "vestibular wall". The lateral wall of the vestibule includes the unsupported soft tissue defined by the nostrils rostrally, the lateral free border of the nasal bone dorsally, the dorsal free border of the incisive bone ventrally, and the intersection of the nasal and incisive bone caudally. In the horse, the dorsal border of the unsupported region can include the dorsal lateral nasal cartilage and, in some species, the ventral border can include the ventral lateral nasal cartilage. Herein, "soft tissue" has its general meaning including skin, muscle, fat, connective tissue or associated integumentary structures.

Also, for purposes herein, the vestibular wall can be divided into at least two portions, a rostral portion being that portion nearest the nostril and a caudal portion being that portion nearest the intersection of the nasal and incisive bone. The region including about the caudal one quarter to one third of the vestibular wall is referred to as the "caudal apex region" and is more fully described below.

It should be noted that an NSD according to the present invention need only engage the lateral free wall of the vestibule, it preferably does not engage the nostrils. Upon visual inspection it will be appreciated that the nostril of the horse can expand to a cross-sectional area that is greater than an unsupported cross-sectional area measured in the nasal cavity in the region of the lateral vestibular wall. However, it is foreseen that support of the nostril could be provided in some circumstances. In the horse, for example, the muscles of the nose and upper lip generally provide significant flaring of the nostril during labored breathing.

The configuration and arrangement of an NSD of the invention is determined by the configuration of the tissue to be supported, the amount of support needed and the unique physiological or anatomical characteristics of the animal. Generally, the unique nasal anatomy of domestic animals necessitates configurations, arrangements or dimensions which are different than that required for a human nose. In addition, hair, sweat and vestibular wall mobility affect the structural arrangement necessary for functionality of an NSD for animals.

In a typical embodiment, a herein disclosed NSD provides support to a portion of the right and left lateral vestibular walls of the animal. Generally, a support device includes a right and left side piece, each including a support layer, which when secured to the nose of the animal are positioned to provide structural support to the right and left lateral vestibular walls. The "right" and "left" side pieces can also be referred to as "first" and "second" or "second" and "first" side pieces. In some embodiments, the support device is bilaterally symmetrical and the side pieces of the device meet at the midline in the midline region of the device. According to this embodiment, when the support device is secured to the nose of an animal, the intersection of the right and left side pieces at the midline preferably overlies or straddles the intersection of the left and right nasal bones and the right and left side pieces overlie the first and second vestibular walls, respectively.

The side pieces and the midline region of an NSD each have a rostral end, a caudal end and a rostral-poll dimension. Because of the size and related anatomical characteristics of the surface area of the vestibular free wall of, for example a horse, to provide sufficient support to benefit the animal, the rostral-poll dimension at the midline region of an NSD of the invention can be substantially equal to or greater than the rostral poll dimension of the side pieces that engage the vestibular free wall. Hence, in one embodiment, the rostral-poll dimension of the midline region is at least as great as the rostral-poll dimension of either of the side pieces. In an alternative embodiment, the rostral- poll dimension of the midline region is greater than the rostral-poll dimension of the right or left side piece.

As used herein, the term "support" refers to reducing the amount of narrowing of the nasal passage that can occur during inspiration or expiration of domestic animals. Accordingly, "support" can include a situation in which there is some drawing in of the vestibular free wall at the rostral nasal passage during inspiration, but less than that which would occur without a device of the invention. "Support" also includes maintaining the position of the external soft tissue over the rostral nasal passage in a neutral position. As used herein, "neutral" refers to a state where the unsupported vestibular tissues are neither drawn into the nasal cavity nor protruding externally relative to a resting position. In some arrangements, "support" also includes maintaining the vestibular free wall in a "distended" outward position relative to the neutral position.

The configuration, arrangement and components of a support device for animals as disclosed herein takes into account the anatomical and physiological characteristics of the vestibular free wall as well as the bony structures defining the borders of the vestibular free wall, unique problems presented in applying the device to an animal as well as equipment used on or around an animal wearing a nasal support device. Moreover, in most large domestic animals, the structural support necessary to support the lateral free wall must also take into account the weight of the tissue supported and the proper leveraging for distributing the weight supported without causing pressure sores or other irritation to surrounding tissues. In addition, due to the muscle control of the upper lip of the horse, supporting the vestibular free wall must also address the active and passive mobility of the muzzle structures.

The size of a device of the invention can vary. Appropriate sized devices will typically correspond with muzzle size which can vary with the body size, breed, age, and sex, of the animal. It is foreseen that smaller size NSDs for young animals, such as calves and foals can be beneficial in treating, for example, upper or lower respiratory ailments. In some embodiments, the rostral-poll midline dimension of an NSD for an average sized adult horse is about 3 to 16 cm, preferably 6-14 cm and the rostral poll dimension of the right and left side is about 3 to 12 cm.

The transverse dimension of an NSD can also vary. The "transverse dimension" is defined as the length of the device from the peripheral edge of one side of the device to the peripheral edge of the second side of the device. The transverse dimension can be approximately equal at the rostral and caudal edge. Alternatively, the transverse dimension can vary in a single device depending if measured, for example, along the caudal edge, the rostral edge, the narrowest part or the widest part or somewhere in between. In one embodiment of an NSD for an average size horse, the transverse dimension at the narrowest part can be about 5-12 cm and about 10-17cm at the widest part.

Typically, an NSD according to the invention includes at least a "support layer" and an "engaging layer". A release liner which is peelably attached to the engaging layer can be used. In some embodiments a "surface layer" can be present to cover the side of the support layer that is away from the nose of the animal when the device is secured to the nose of the animal. Some embodiments can also include a carrier layer which can be removably attached to the surface layer to facilitate handling and proper placement of the support device on an animal's nose. The exterior color of the surface layer is preferably selected to reduce the likelihood of the device causing interference with equipment used for determining results of a particular competitive event.

The support layer of the device provides the majority of the support for the vestibular free wall of the nasal passage of an NSD. Generally, support is provided in the support layer through the use of one or more "lift members." As used herein a "lift member" can be prepared from any suitable material which provides the desired support to the vestibular free wall. Examples of suitable materials for a lift member include thermoplastic resins, thermoset resins, shape memory metals, alloys, leather, etc. The lift member can be a unitary open mesh or solid material. One example of a preferred material for a lift member is a biaxially oriented polyester such as MYLAR® available from DuPont Films, Wilmington DE. Other suitable materials for a lift member are disclosed in U.S. Patent No. 5,913,873 and International Patent Publication WO 98/47451 which are incorporated herein by reference.

In some embodiments, the lift members are of a generally uniform thickness throughout their length and width. The thickness of the lift members will typically be selected based on the support needed, and is generally the same throughout. However, the lift members can also vary in thickness in different regions of the device. In addition, a lift member need not be the same width throughout its length. That is, a lift member can be wider at the lateral ends of the lift member and narrower near the midline region. Alternatively, a lift member can be wider in the region that will lie over the midline region of the nose and narrower on the ends.

Suitable thickness for a lift member prepared from a polyester such as MYLAR® for an adult large animal such as a horse is about .008 to about .020 inches. In one preferred embodiment, the thickness of a support member for an average size adult horse is about .014 inches.

The support layer can include one or more lift members. The lift members can be positioned parallel to the transverse axis of the device and extend partially or completely to the lateral edges of the device. Three to six lift members are preferred for some animals such as a horse. When more than one lift member is used, the width, length and spacing of the lift members can vary based on the overall dimensions of the particular device. Also, the length of the individual lift members can vary in a single device so as to traverse some or all of the dorsal-ventral dimension of the vestibular free wall. Preferably, the transverse length dimension of a lift member is sufficient to traverse the midline of the animal's nose and extend to the right and left side pieces beyond the dorsal lateral nasal cartilages to support the right and left vestibular free walls. In some embodiments, the lift members can extend beyond the ventral edge of the vestibular free wall to a point lateral to the incisive bone. Generally, the lift members provide a "lift" effect on the vestibular free wall to reduce drawing of the vestibular free wall into the nasal passage during respiration. However, if the lift members extend beyond the ventral edge of the vestibular free wall to the lateral aspect of the incisive bone, the incisive bone can act with the lift members to "stent" the vestibular free wall and facilitate the reduction of the drawing of the vestibular wall into the nasal cavity that is provided by the lift members. This may be particularly advantageous in large animals during labored breathing.

When using multiple lift members, the spacing between individual lift members can affect the adherence and overall functioning of the device. When two or more lift members are used, the width of the lift members and the spacing between lift members are selected for the NSD to provide the desired support to the vestibular wall with sufficient flexibility to reduce the chance of irritation due to localized pressure at leveraging points on the animal's nose. Use of multiple lift members advantageously provides for torsional flexibility of the device which facilitates function and reduces the likelihood of disengagement of the device when subjected to the unique mobility of an animals vestibular tissues. In one embodiment of an NSD for an average size adult horse, the length of the lift members can be about 4-18 cm, preferably about 9-13 cm, the width can be about 0.2 to 2 cm and the spacing between lift members about 0.2 to 2 cm, preferably about 0.3 to 1.0 cm.

In one embodiment, the lift member can be a single member of a previously described solid or open mesh material that is shaped to support or stent a single vestibular wall. That is, the outer contours of the lift member are configured to follow the peripheral margins of the vestibular wall. Preferably, the perimeter edge of the lift member is extended to allow the lift member to overlap the borders of the vestibular wall by about 0.2 - 2 cm. According to this embodiment, the lift member can include an engaging layer and optionally a surface or pad layer. Preferably, the engaging layer extends beyond the perimeter of the lift member to enhance adherence to an animal's nose. However, rather than connecting at the midline region of the nose, this embodiment of an NSD comprises two separate pieces, that act as a stent for each vestibular wall.

An NSD preferably includes an engaging layer. The engaging layer provides for securing an NSD to the animal's nose. If no surface layer (described below) is present and individual lift members are used, the engaging layer can also provide for maintaining the unity of the device. Typically, the engaging layer can secure the NSD to the nose by use of an adhesive. Other invasive forms of engaging to the nose, such as suturing, are possible but not desired. Preferably, the adhesive is biocompatible and provides minimal or no contact irritation when applied to the external tissues of an animal.

Suitable materials for the adhesive of the engaging layer are single or double coated medical tape, transfer adhesives, liquid adhesives, pressure sensitive adhesives (PSA), etc. A release liner is preferably applied to the adhesive of the engaging layer to cover the adhesive surface until the support device is applied to an animal. In some embodiments, the release liner can comprise one or more sections which can be selectively removed from the engagement layer to facilitate positioning of the support device on the nose. Examples of suitable adhesive systems include No. 1509 double sided medical tape, No. 9942 Hydrocolloid Skin Protective Adhesive and No. 1524 transfer adhesive available from 3M Co., St. Paul, MN. One presently preferred adhesive is Dermamed DM-2009, available from Dermamed, 381 Geneva Avenue, Pallmadge, OH 44278.

The NSD can include a surface layer. The surface layer is the layer farthest from the soft tissues of the animal and is visible when the device is applied to the animal's nose. Thus, one side of the surface layer faces the soft tissue on the animal's nose and a second side faces away from the nose. The side of the surface layer closest to the soft tissue of the animal can include an adhesive to adhere the surface layer to the support layer, to the side of the engaging layer that may be exposed between lift members, or to a pad layer if used. The surface layer can provide additional support to the vestibular wall and help maintain unity of the components of an NSD. A suitable surface material can be breathable or non-breathable and typically includes a biocompatible adhesive. An example of a breathable material suitable as a surface layer is No. 1533 available from 3M Inc., St. Paul, MN. One preferred non-breathable surface material is No. 9906T non-woven medical tape available from 3M Co., St. Paul, MN. Another example of a suitable surface layer is No. 9910 non-woven medical tape available from 3M Co., St. Paul, MN.

The surface layer can include an ornamental design color, pattern, logo etc. if desired. Alternatively, an ornamental veneer layer can be applied to the exposed surface of a surface layer or support layer. The color of the surface layer is preferably selected so as to reduce glare which can interfere with photographs taken to determine the outcome of a performance event such as a finish line photograph taken in a horse race. Examples of suitable colors which cause reduced glare are dark colors such as black, dark blue, dark green, dark gray, dark brown, etc.

The engaging layer may extend only to the peripheral extent of the lift members of the support layer. Alternatively, the periphery of the engaging layer can extend beyond the peripheral extent of the lift members of the support layer (i.e., laterally, rostrally and caudally). In some preferred embodiments, the periphery of the engaging layer can extend beyond the support layer to provide improved engagement of the support device to the animal's nose. In one such embodiment a region of about 0.5 cm to 4 cm, preferably about 1.0 - 2.0 cm of engaging layer extends beyond the lateral extent of the lift members of the support layer and 1.0-3.0 cm beyond the rostral-caudal extent of the support layer. The surface layer typically extends the same distance beyond the lift members as does the engaging layer. If present, the surface layer typically has the same perimeter dimensions as the engaging layer.

Generally, the overall thickness of the device is uniform. Some variation in thickness can occur due to differences in thickness of those regions of the device including the support members and those regions having spacing between support members.

The inventors also recognize that single or multiple lift members without a unifying layer (e.g., surface layer or engaging layer) can be used. According to this embodiment, an engaging layer, such as a previously described adhesive, can be applied to the lift member. One or more lift members can then be applied directly to the animals nose. While this embodiment may address the unique physiological and anatomical aspects of an animals nose as disclosed herein, the application and removal of the support members will be cumbersome. In addition, the support provided by the surface layer or engaging layer in the regions between the lift members will be lost.

A support device according to the invention can also include a "carrier layer". The carrier layer can be removably adhered to the side of the surface layer away from the animal's nose. The carrier layer can be made from any suitable material including paper, metal foil, plastic, cardboard, etc. The carrier layer is preferably adhered to the surface layer using an adhesive system which provides a peel resistance which is less than the peel resistance between the adhesive of the engaging layer and the animal's nose when the support device is adhered to the animal's nose. Suitable adhesive systems for adhering the carrier layer to the surface layer are known and disclosed in, for example, U.S. Patent Nos. 3,691,140; 4,994,322; 5,266,402; 5,502,109; and 5,719,247. The entire disclosure of each of these patents is incorporated herein by reference. One preferred adhesive is No. 9425 available from 3M Co., St. Paul, MN. This adhesive system is a double side tape wherein a first side of the tape (applied to the carrier layer) has a greater peel resistance than the second side of the tape (applied to the surface layer) and the peel resistance of the second side of the tape is less than the peel resistance of the adhesive between the engaging layer and the animal's nose.

The perimeter edge of the carrier layer can follow the perimeter edge of the surface layer of the support device or the perimeter edge of the carrier layer can extend beyond the perimeter edge of the surface layer. Alternatively, portions of the carrier layer can extend beyond the perimeter edge of the surface layer and other portions follow the perimeter edge or not extend to the perimeter edge of the surface layer. Extending the perimeter edge of the carrier layer beyond the perimeter edge of the surface layer provides a region of the carrier layer which can be grasped for handling or removing the carrier layer from the surface layer without contacting the adhesive of the engaging layer. Alternatively, the carrier layer can be slit to provide an internal edge to grasp for removing the carrier layer from the surface layer. In a preferred embodiment, the perimeter edge of the carrier layer can be configured to provide a guide for positioning of the support device on an animal's nose.

Thus, after removal of some or all of the release liner from the adhesive of the engaging layer, the carrier layer can be grasped during application of the device without contacting the adhesive of the engaging layer. Once engaged to the nose, the carrier layer can be peelably removed from the support device. The carrier layer can also be marked with instructions for proper positioning and orientation of the support device to assist the person applying the device to the animal.

In another embodiment of the invention, a support device can comprise two separate pieces, one for supporting a portion of each of the right and left vestibular wall For example, only the caudal aspect of each of the vestibular walls may be supported by the device. According to one such embodiment, the support device can extend from the nasal bone to the incisive bone, across the "caudal apex region" of the vestibular wall near the intersection of the nasal and incisive bones. In an alternative embodiment, the support device can extend rostrally over the caudal apex of the vestibular wall from a location caudal to the intersection of the incisive and nasal bones. In another embodiment, the support device can extend from the nasal bone to the incisive bone, across the caudal apex of the vestibular wall, and extend caudally over the intersection of the incisive and nasal bones.

Examples of support devices and methods of the invention will now be further described by reference to the following illustrated embodiments.

### Detailed Description of Illustrated Embodiment

Anatomical reference points and embodiments of an NSD according to the invention will be described in detail with reference to the drawings using the horse as an example. Like reference numerals represent like parts and assemblies throughout the several views. Reference to the drawings is not intended to limit the scope of the invention to the illustrated embodiments.

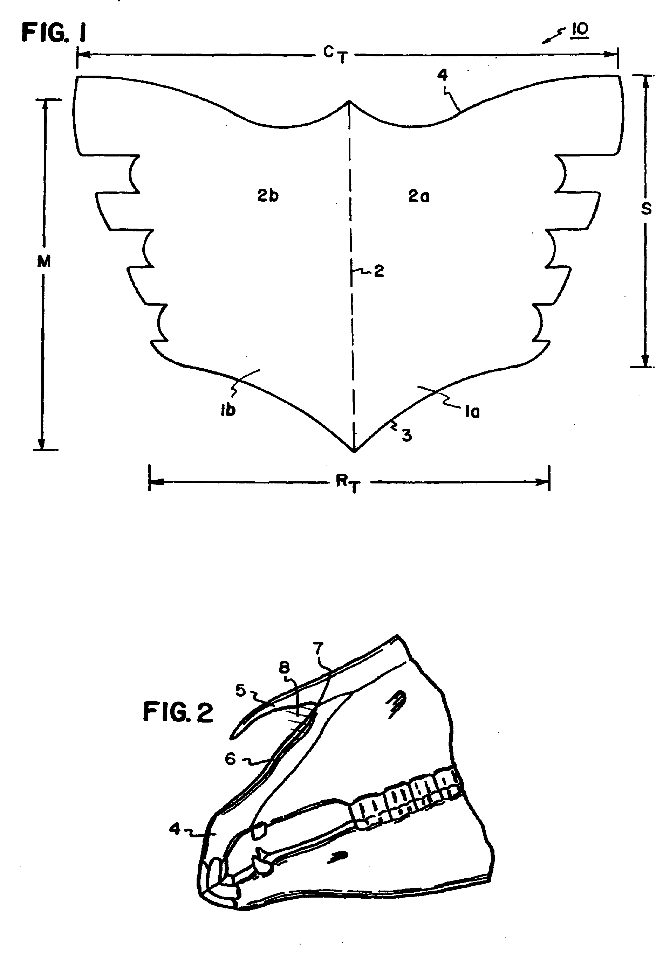

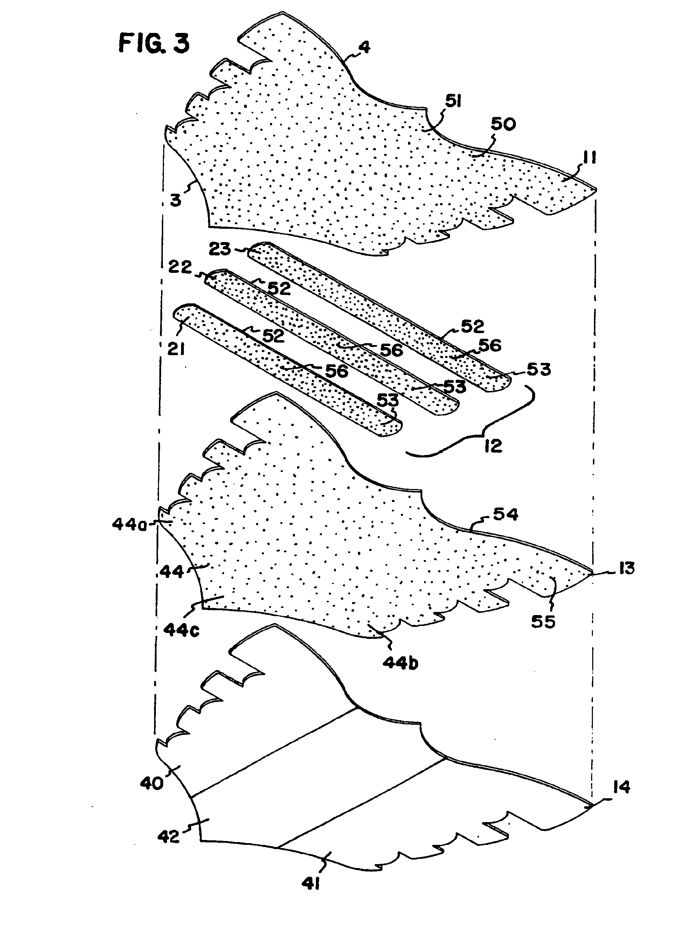

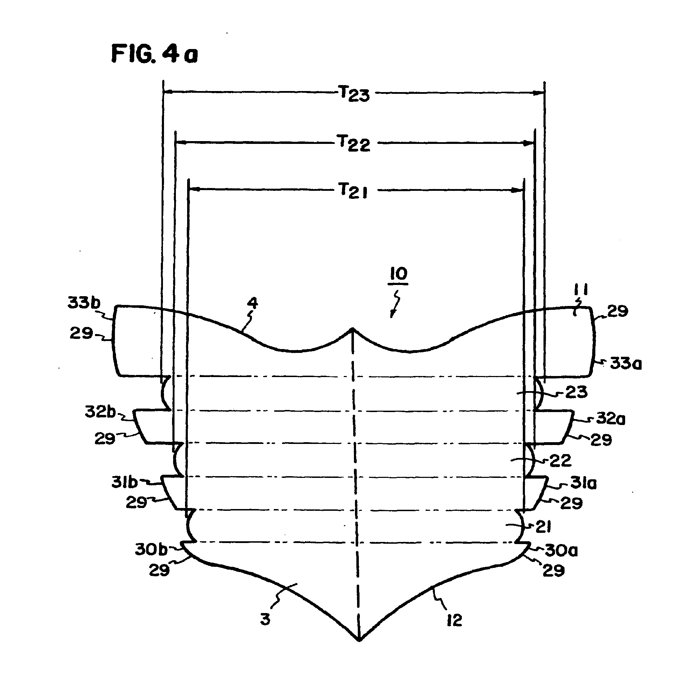

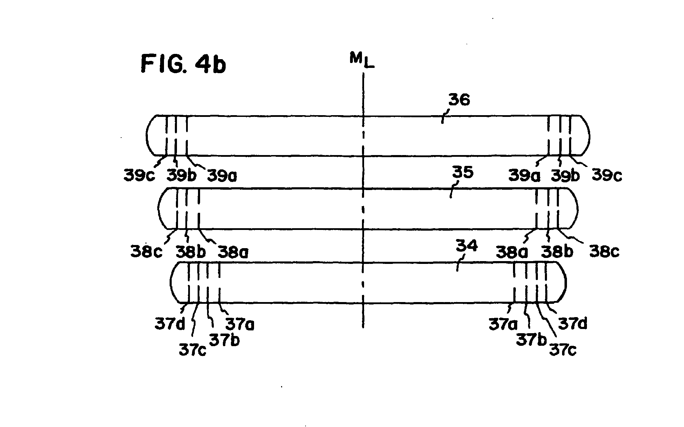

FIG. 1 is a top plan view of the configuration of one embodiment of a nasal support device (NSD) **10** according to the invention. Dimensions which can be used to characterize an NSD are shown wherein letters followed by a subscript "T" are transverse dimensions. Thus, **C**_{**T**} is the caudal transverse dimension, **R**_{**T**} is the rostral transverse dimension, **S** is the rostral-poll dimension of the side piece and **M** is the rostral- poll dimension at the midline of the device. The bottom plan view of NSD **10** is substantially identical to the top plan view of FIG. 1 except that the support layer, shown in FIGs. 3 and 4, may be visible in the bottom plan view.

The NSD **10** includes a first side piece **1a** and a second side piece **1b** that intersect at the midline **2** of the midline region **2a** and **2b.** In use, the rostral end **3** is oriented towards the apex of the animal's nose and the caudal end **4** is oriented towards the eyes of the animal. In the embodiment of FIG. 1, the midline rostral-poll dimension **M** is at least equal to the rostral-poll dimension **S** of side pieces **1a** and **1b.** In some embodiments, the rostral-poll dimension of the NSD at the midline **2** can be greater than the rostral-poll dimension **S** of the first or second side pieces **1a, 1b.** It will be appreciated that in the illustrated embodiment ,the rostral transverse dimension **R**_{**T**} is less than the caudal transverse dimension.

Bony anatomical structures which surround the vestibular free wall which is supported by a support device of the invention are described with reference to FIG. 2 which is a profile view of the bony anatomy of the rostral region of the horse's head. A more complete discussion of the relevant anatomy is disclosed in U.S. Patent No. 5,913,873, the entire disclosure incorporated herein by reference. Briefly, the lateral free wall of the vestibule is defined dorsally by the lateral aspect of the nasal bone **5,** ventrally by the incisive bone **6,** and caudally by intersection **7** of the nasal **5** and incisive **6** bones. The rostral aspect of the vestibular free wall is bordered by the nostril (not shown).

The shaded area **8** in FIG. 2 depicts the approximate area underlying the "caudal apex region" of the vestibular wall. In some embodiments, a support device of the invention may be configured to support only the caudal apex region of the vestibular wall to facilitate air flow through the nasal passages. The caudal apex region is nearest the nasal valve region of the nasal passages which is particularly vulnerable to narrowing under certain conditions.

FIG. 3 is a bottom exploded view of NSD **10** illustrating a surface layer **11,** support layer **12,** engaging layer **13** and release liner **14.** A carrier layer is not shown in this view. FIG. 4a is a top plan view of the NSD **10** of FIGs. 1 and 3 showing rostral **21,** intermediate **22** and caudal **23** lift members in phantom lines. In this embodiment, the transverse length **T**_{**21**}**, T**_{**22**}**,** and **T**_{**23**} of lift members **21, 22,** and **23,** respectively, increases from the rostral end **3** to the caudal end **4** of NSD **10.** In addition, lateral engagement extensions **29,** comprise rostral **(30a, 30b),** rostral/intermediate **(31a, 31b),** caudal/intermediate **(32a, 32b)** and caudal **(33a, 33b)** lateral engagement extensions. Lateral engagement extensions **29** are comprised of surface layer **11** and engaging layer **13** which extend beyond the transverse length (i.e., lateral extent) of lift members **21, 22,** and **23** to facilitate engagement of NSD **10** to the nose of the animal. The functional aspects of the lateral engagement extensions can be embodied in various overall device appearances.

FIG. 4b is a top view of an alternative embodiment of the lift members of NSD **10.** In this view, only lift members **34, 35, 36** are shown and they are in the same relative position as shown in phantom lines in FIG. 4a. At the lateral extent of each of lift members **34, 35, 36,** there are pairs of notches **37a-d, 38a-c, 39a-c** which can penetrate through a partial or full thickness of each of the lift members **34, 35, 36.** In the illustrated embodiment, four pairs of notches **37a-d** are present at the lateral end of rostral lift member **34** and three pairs **38a-c, 39a-c** are present at the lateral end of intermediate lift member **35** and caudal lift member **36.** It will be appreciated that in this embodiment, the most lateral notches **37d, 38c, 39c** of each lift member traverse a greater portion of the width of the lift members (i.e., smaller unnotched region between notches of a pair) than do notches nearer to midline **M**_{**L**} of lift members **34, 35, 36.** The number of notch pairs can be varied. Typically, if additional notch pairs are added, they are added nearer the midline **M**_{**L**} region of the lift member.

The effect of notches which traverse an increasing portion of the width dimension of the lift member from nearest the midline **M**_{**L**} to the lateral end is to gradually decrease the peel force exerted on the engaging layer between the support device and the animal's nose and convert it to a shear force to facilitate engagement of the NSD **10** to the animal's nose.

Referring to FIG. 3, release liner **14** can comprise a single section or have a single or multiple slits to make a multiple section release liner. Suitable release liners for use with an adhesive of the engagement layer are known. In one preferred embodiment, illustrated in FIG. 3, release liner **14** comprises three components, a first lateral piece **40,** a second lateral piece **41** and an intermediate piece **42.** According to this embodiment, when applying NSD **10** to an animal's nose, intermediate piece **42** can be removed first and NSD **10** positioned over the nasal bones **5,** and the midline region **44c** of adhesive **44** of engagement layer **13** lightly engaged to the skin over the nasal bones. Some repositioning can be performed before the lateral aspects **44a** and **44b** of the adhesive **44** is exposed. Once the proper final position of the NSD **10** is determined, first lateral piece **40** and second lateral piece **41** of release liner **14** can be removed and the lateral aspects **44a** and **44b** of adhesive 44 secured to the animal's nose.

In the embodiment of FIG. 3, the bottom side **50** of surface layer **11** (i.e., the side towards the animal's nose when in use) includes an adhesive layer **51** to adhere the surface layer **11** to the top side **52** of lift members **(21, 22, 23)** and to the top side **54** of engaging layer **13.** The bottom side **53** of lift members **(21, 22, 23)** can include an adhesive **56** to adhere the lift members **(21, 22, 23)** to the top side **54** of the engaging layer. The bottom side **55** of engaging layer **13** includes adhesive **44** to adhere the device to the animal's nose. Each of the adhesives of NSD **10** can be a coated medical tape, transfer adhesive, liquid adhesive, PSA, etc. In one preferred embodiment, the surface layer **11** is 9910 black non-woven medical tape available from 3M Co., St. Paul, MN, the lift members **21, 22, 23** are MYLAR® available from DuPont Films, Wilmington, DE, the engaging layer **13** is DM-2009, available from Dermamed, Pallmadge, OH 44278 and the release liner is DM-2009 release liner, also available from Dermamed.

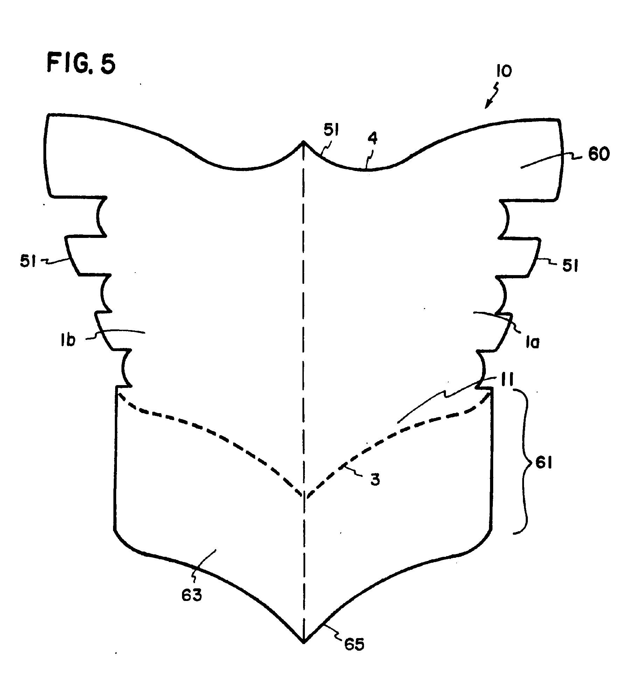

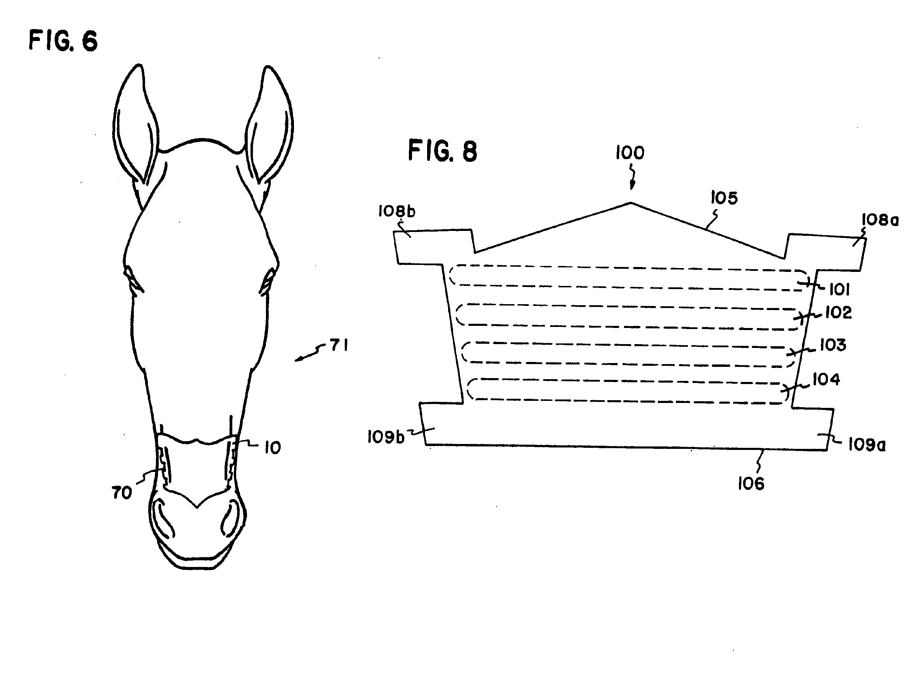

In some embodiments, an NSD can include a carrier layer. FIG. 5, is a top view of an NSD **10** including a carrier layer **60** which is releasably adhered to the surface layer **11.** In this embodiment, carrier layer **60** follows the perimeter edge **51** 1 of surface layer **11** except at the rostral end **3** of the NSD **10.** At the rostral end **3,** the carrier layer **60** includes a rostral extension **61.** Rostral extension **61** provides a grasping portion **63,** for handling NSD **10** with reduced likelihood of contacting the adhesive **44** of engagement layer **13,** if the release liner has been removed. In addition, in the embodiment of a carrier layer **60** of FIG. 5, the rostral extension **61** is configured to provide an alignment guide for proper positioning of the NSD **10** on an animal's nose. Specifically, by aligning the rostral edge **65** of carrier layer **60** at the apex of a horse's nostrils, the side pieces **1a** and **1b** will be properly aligned over the lateral vestibular walls. FIG. 6 is a front view of an NSD **10** secured to the nose **70** of a horse **71.**

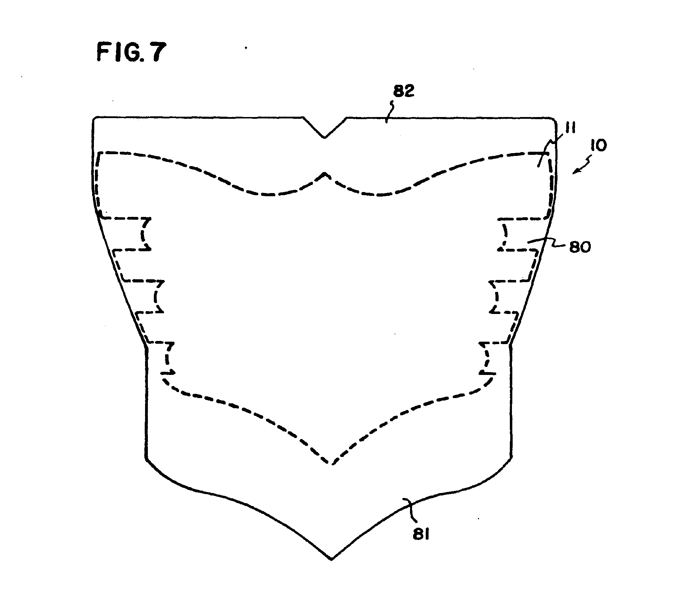

FIG. 7 is an NSD **10** having an alternative embodiment of a carrier layer **80** releasably adhered to the surface layer **11.** As with carrier layer **60** of FIG. 5, carrier layer **80** includes a rostral extension **81.** In addition, carrier layer **80** also includes a caudal extension **82** to facilitate handling and positioning of the device **10.** In other embodiments, the carrier layer could be configured to provide lateral extensions or a perimeter extension around the entire device.

A carrier layer and release liner as disclosed herein can also be used with human nasal support dilators as well as with animal nasal support devices as disclosed in, for example, U.S. Patent No. 5,913,873 and co-pending applications U.S. Serial Nos. 09/018,603 and 09/264,464, the entire disclosures of which are incorporated herein by reference.

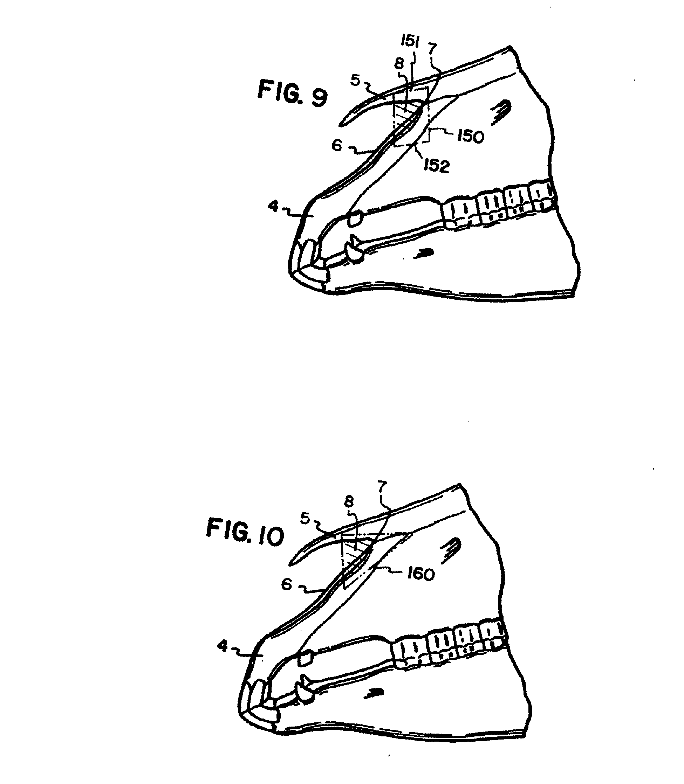

FIG. **8** is a top view of an alternative embodiment of an NSD **100** according to the invention. Four lift members **101, 102, 103** and **104** are illustrated in phantom lines. It will be appreciated that in this embodiment, lift member **101** at the rostral end **105** of NSD **100** has the longest transverse dimension and lift member **104** at the caudal end **106** has the shortest transverse dimension. Lateral engagement extensions **108a, 108b** and **109a, 109b** are also present for purposes previously discussed.

FIGs. 9 and 10, illustrate an alternative embodiment of a support device and method of the invention. For exemplary purposes, the following description will be made with illustrations of a support device applied to the skeletal anatomy of a horse to appreciate the relative positions of the anatomy and the support provided.

The embodiments of FIGs. 9 and 10 provide localized support to the caudal apex region of the vestibular wall. FIG. 9 illustrates the positioning, relative to shaded area 8, of a support device **150** when applied to the caudal apex region of the vestibular wall. As illustrated, support device **150** has a dorsal edge **151** which overlies a portion of nasal bone **5** and a ventral edge **152** which overlies a portion of incisive bone **6.** The amount of dorsal edge **151** and ventral edge **152** which overlies nasal bone **5** and incisive bone **6** is preferably at least about 0.5 cm and typically about 1.0 to 3.0 cm.

FIG. 10 illustrates an alternative embodiment for supporting the caudal apex region of the vestibular wall. In this embodiment, support device **160** extends caudally beyond the intersection **7** of nasal bone **5** and incisive bone **6.** Preferably, support device **160** extends about 0.5 cm to about 5.0 cm caudal to intersection **7.** Support device **160** also extends dorsally and ventrally over nasal bone **5** and incisive bone **6,** respectively, as described for support device **150.**

It will be appreciated that although support device **150** is illustrated as a rectangle and support device **160** as a triangle, other shapes, including squares, circles, ovals, octagons, etc., can be used to provide function according to this aspect of the invention. In addition, support devices **150** and **160** can include a carrier layer, surface layer, support layer, engaging layer and release liners as described for other embodiments of the invention. The support layer can comprise one or more lift members made of previously described materials.

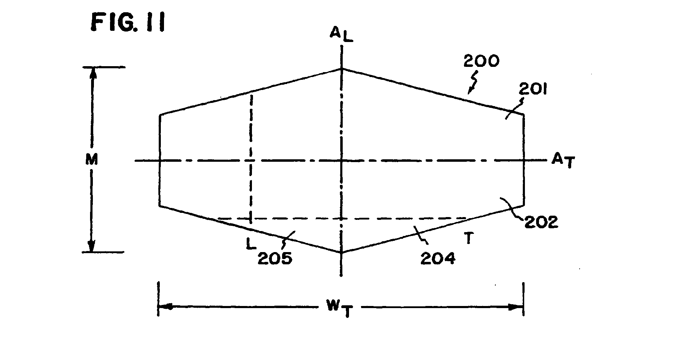

In another embodiment, an NSD according to the invention provides a bilaterally symmetrical NSD across two dimensional axes of the device. Referring to FIG. 11, a general configuration for an NSD according to this embodiment is diagrammatically illustrated. As illustrated, NSD **200** has a transverse axis **A**_{**T**} through the widest transverse dimension **W**_{**T**}**.** NSD **200** also has a longitudinal axis **A**_{**L**} through the longest rostral-caudal dimension **M.** Thus, although other longitudinal dimensions **L** and transverse dimensions **T** are present, the greatest dimension in either the longitudinal or transverse direction is at the axes.

According to this embodiment, the NSD is bilaterally symmetrical on opposing aspects of transverse axis **A**_{**T**}**.** That is, the upper surface **201** of NSD **200** looks substantially identical to the bottom surface **202.** In addition, the NSD is bilaterally symmetrical on opposite sides of longitudinal axis **A**_{**L**} such that the right surface **204** is substantially identical to the left surface **205.** It will be appreciated that each axes **A**_{**T**} and **A**_{**L**} bisects the device into bilaterally symmetrical halves in their respective dimensions.

The general appearance of NSD **200** of FIG. 11 can include any of the structural features or components of other NSDs disclosed herein, but, in addition to other advantages, this configuration provides for ease of application and reduced likelihood of malfunction due to improper positioning. Specifically, the device provides structural features in a configuration which can be placed on the nose in the position illustrated in FIG. 11 or rotated 180° therefrom.

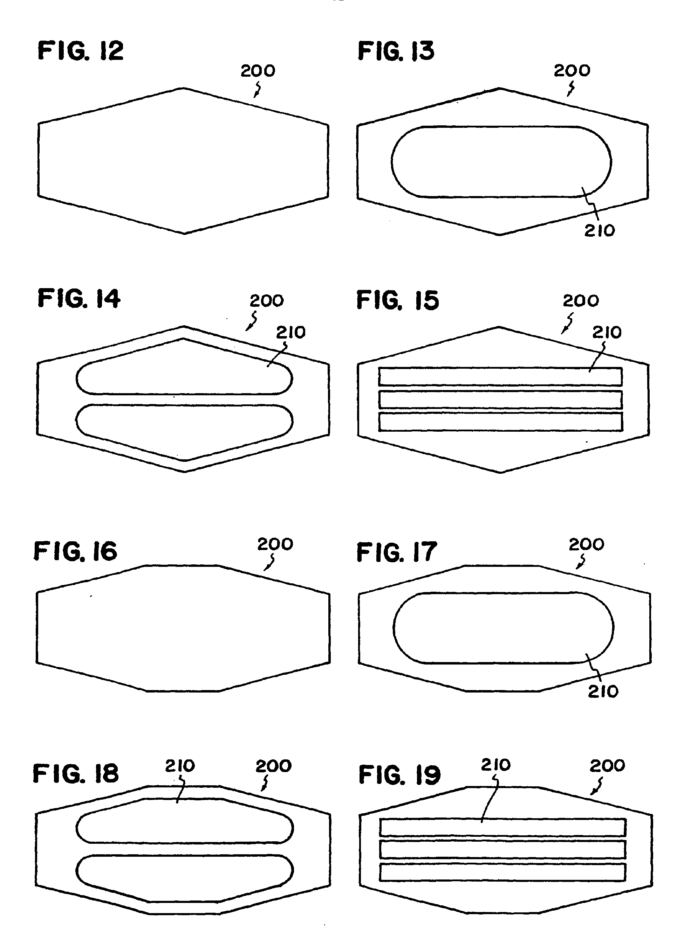

In addition, the two axes bilaterally symmetrical devices can include all structural aspects, components, or functional features described above for other NSD embodiments, but within varying appearances as illustrated in FIGs. 12-37 as described below.

FIG. 12 illustrates a top side view of one appearance for the configuration of a two axes bilaterally symmetrical NSD as described above with reference to FIG. 11. FIG. 13-15 illustrate a bottom side view of the embodiment of FIG. 12 with varying numbers of support members **210** as described earlier. An intermittent adhesive pattern can be used as described. Holes may also be placed through the device, preferably not located in or through the lift members.

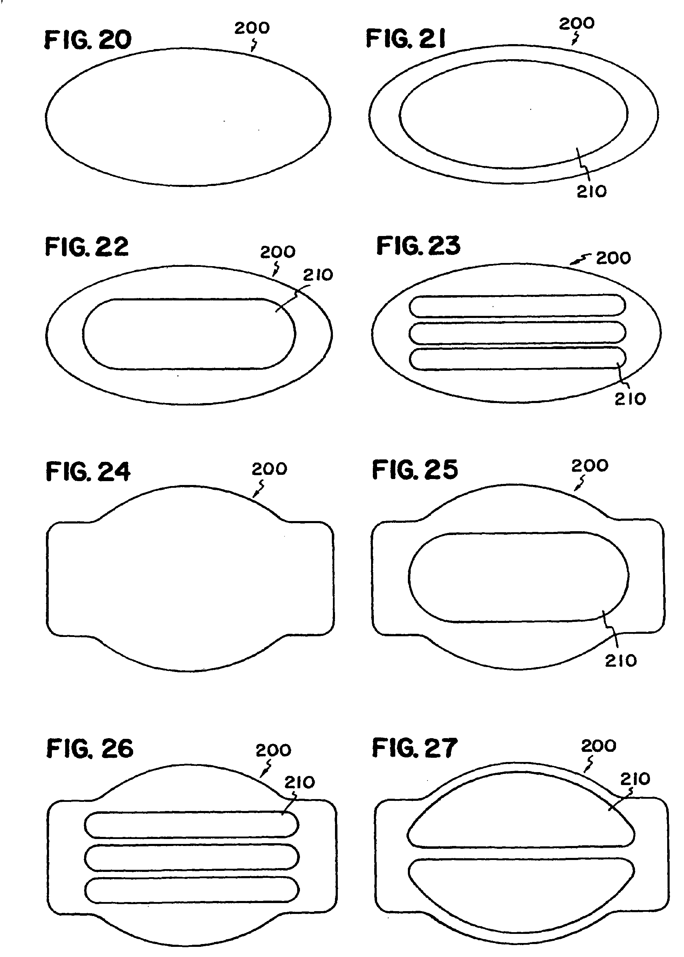

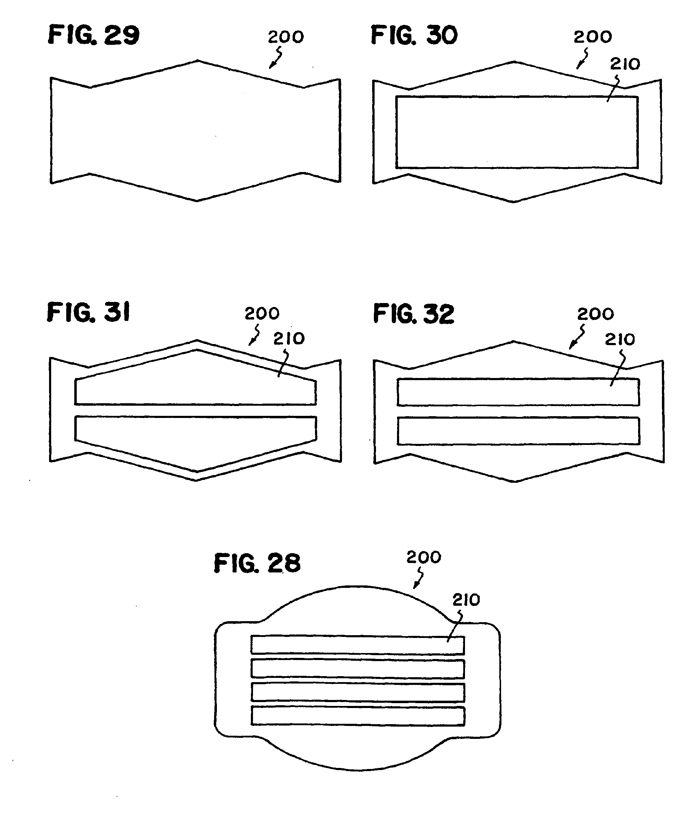

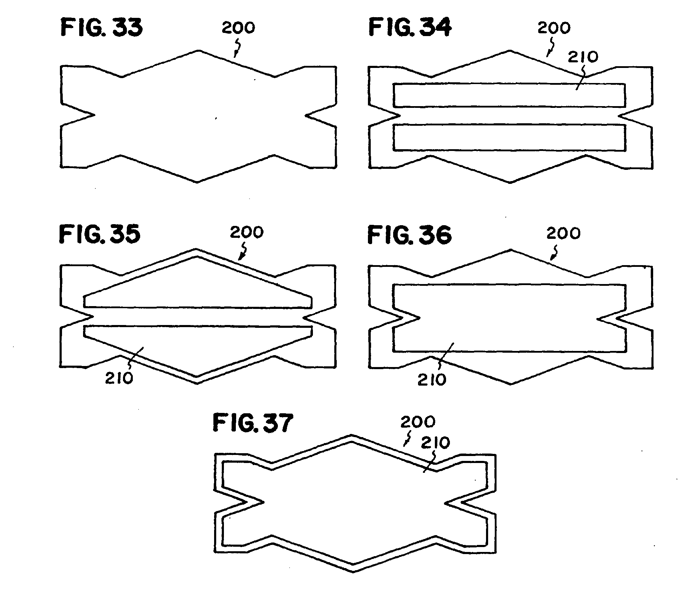

FIG. 16 is a top side view and FIGs. 17-19 are bottom side views of an alternative appearance of an NSD containing the configurational features described. FIG. 20 is a top side view and FIGs. 21-23 are bottom side views of an alternative appearing embodiment. FIG. 24 is a top side view and FIGs. 25-28 are bottom side views of another alternative appearing embodiment. FIG. 29 is a top side view and FIGs. 30-32 are bottom side views of yet another alternative appearing embodiment. FIG. 33 is a top side view and FIGs. 34-37 are bottom side views of another alternative appearing embodiment of an NSD according to the invention.

Thus, each of the foregoing embodiments illustrated in FIGs. 12-37 can include some or all of the structural features or arrangements of other NSDs disclosed herein but with different ornamental appearances. The side view of each of the embodiments is substantially void of any ornamental features. In addition, the lift members can be of the same number, size, shape, material, material thickness, etc. as previously described. In addition, construction features, component layers, adhesive features, holes, intermittent adhesive patterns, etc. can be used.

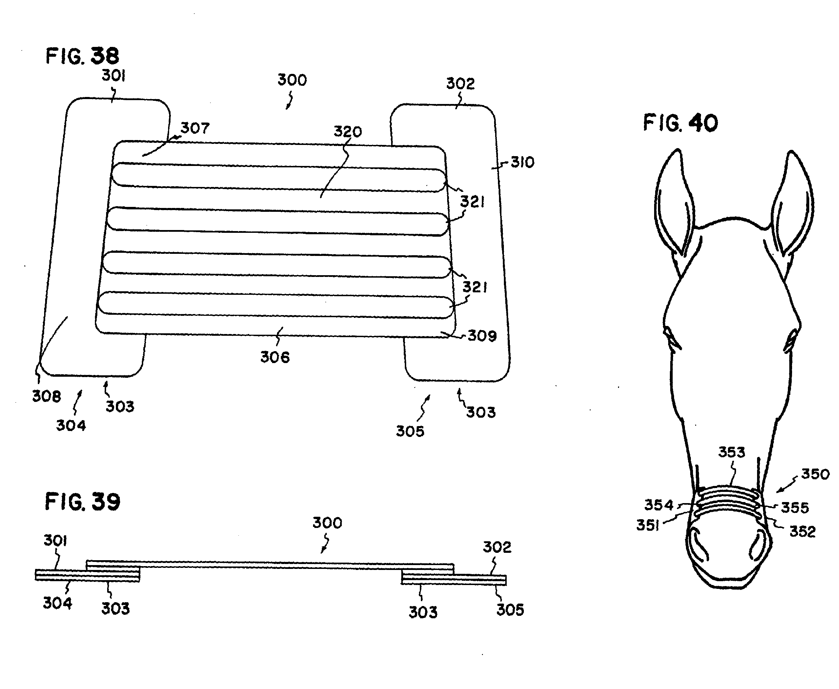

In another embodiment, an NSD **300** according to the invention can be provided in component parts, some of which are reusable. FIG. 38 is a top plan view of one such embodiment and FIG. 39 is an end-on view of the embodiment of FIG. 38 looking from the rostral aspect **R** According to this embodiment, NSD **300** includes a first side piece **301** for engaging to a first lateral vestibular wall and a second side piece **302** for engaging a second lateral vestibular wall. An engaging layer **303** including adhesives and adhesive systems as described earlier can be applied to the bottom surfaces **304** or **305** of side pieces **301** and **302,** respectively, for adhering the side pieces to the lateral vestibular walls. Bridge piece 306 is configured to traverse the animal's nose and attach at a first lateral region **307** to the first top side **308** of first side piece **301** and attach at a second lateral region **309** to a second top side **310** of second side piece **302.** The bridge **306** can attach to the side pieces **301** and **302** using known systems such as velcro, buckles, zippers, snaps, hooks, hook and loops, snap-rings, clips or other similar attachment providing for reusable attachment of bridge piece **306** to the side pieces. The side pieces 301 and **302** will typically be replaced after each use.

It will be appreciated that bridge piece **306** includes a surface material **320** which keeps individual lift members **312** in a fixed arrangement relative to one another. The components of the surface layer and lift members can be as previously described. Alternative materials consistent with the functional requirements for an NSD of the invention can also be used. One, two, three, four or more lift members **321** can be used as needed. Holes may also pass through the bridge piece **306** which preferably do not pass through lift members **321.**

Side pieces **301** and **302** may or may not include lift members and also may or may not include holes. If used, one or more lift members can be used which are oriented parallel, perpendicular or oblique to the lift members of bridge piece **306.**

FIG. 40 illustrates another embodiment of an NSD **350** having a first side piece **351** and a second side piece **352** substantially as described for NSD **300** above. However, in contrast to bridge piece **306,** the bridge piece of embodiment **350** comprises at least two, typically three and optionally more, independent lift members **353.** Each lift member can attach at its lateral region **354, 355** to side pieces **351** and **352** as described for NSD **300.**

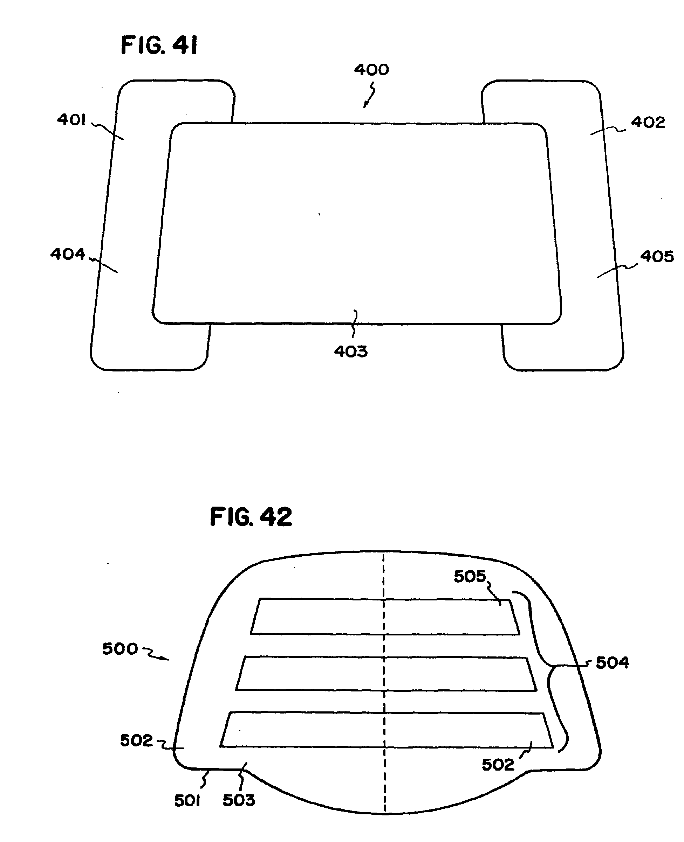

FIG. 41 illustrates another embodiment of an NSD **400** having a first side piece **401** and a second side piece **402** substantially as described for NSDs **300** and **350.** However, bridge piece **403** comprises a single unit piece which traverses the nose and attaches at its lateral edges **404** and **405** to first side piece **401** and second side piece 402 as described above. Hence, for each of the NSD embodiments **300, 350** and **400,** the first and second side pieces can be discarded after use and bridge pieces **306, 353** and **403** can be reused. With each use, the operator can apply the lateral regions to the first and second side pieces with a selected amount of tension for a desired amount of support for the lateral vestibular wall.

The present invention also provides reusable nasal support devices for animals. The term "reusable" refers to a nasal support device which can be applied to an animal, removed and subsequently reapplied to the same or a different animal. A single use of a nasal support device is typically considered in use from the time of adhesion to an animal's nose until removal of the device from the nose.

In one embodiment, a reusable nasal support device can be prepared by application of a reuse engaging layer to the spent engaging layer of a previously used nasal support device. As used herein, "spent engaging layer" refers to the engaging layer used to adhere a nasal support device to an animal after the nasal support device has been removed from the animal. The "reuse engaging layer" provides an adhesive layer for reattaching the nasal support device to an animal's nose after an earlier engaging layer is spent. Nasal support devices which can be used according to this embodiment of the invention include, for example, any of the nasal support devices for animals disclosed herein or in the above recited patents and patent applications. Suitable reuse engaging layers include, for example, double-sided tape or transfer adhesives such as No. 1524 transfer adhesive available from 3M Co., St. Paul, MN.

According to this embodiment, subsequent to application of a nasal support device to an animal, the nasal support device can be removed from the animal and a reuse engaging layer applied to the spent adhesive surface of the engaging layer which previously was in contact with the nose of the animal. The reuse engaging layer is applied to the previously used nasal support device after removal from the animal. However, it will be appreciated that if the engaging layer becomes non-functional (spent) due to the adhesive surface being spent as a result of adhesion or other contact with something other than the nose of an animal, nonetheless, the reuse engaging layers and methods disclosed herein can be used for preparing a reusable nasal support.

When a double-sided tape is used, the adhesive of each side of the tape may be the same or different. For example, one side of the double-sided tape can include an adhesive which provides a high peel resistance from the engaging layer of the previously used nasal support device and the second side of the double-sided tape can include an adhesive which provides an appropriate peel resistance from the animal's nose when applied to the nose. In general, the reuse engaging layer can be applied to a used nasal support device immediately after removal of the nasal support device from an animal. In an alternative embodiment, the nasal support device can be removed from the animal and a period of time allowed to pass before application of the reuse engaging layer applied to the nasal support device. In one embodiment, the reuse engaging layer is not applied to the used nasal support device for at least three hours, typically at least 12 hours and in a preferred embodiment at least 24 hour after removal of the used nasal support device from an animal.

It is foreseen that a nasal support device can be used multiple times and thus include a plurality of reuse engaging layers applied to the nasal support device with or without a reuse of the nasal support device between application of each subsequent reuse engaging layer.

It is also foreseen that in an alternative embodiment, rather than using a transfer adhesive tape or double-sided tape, a single-sided tape can be used. According to this embodiment, one side of the single-sided tape includes an adhesive for adhering the used nasal support device to an animal's nose. The second side of the single-sided adhesive tape does not include an adhesive. Thus, a liquid adhesive can then be applied to the engaging surface of the nasal support device or to the non-adhesive side of the single-sided tape, or both, and the single-sided tape mounted onto the spent engaging layer of the nasal support device. The adhesive side of the single-sided tape faces away from the spent engaging layer.

Preferably, the configuration of a single or double-sided tape or transfer adhesive is pre-cut to substantially the same shape as that of the nasal support device on which the reuse engaging layer will be applied. For example, in a preferred embodiment of a reuse engaging layer for a device as shown in FIG. 3, the reuse engaging layer would be shaped substantially as shown for surface layer 11 or engaging layer 13. However, the configuration of the reuse engaging layer can alternatively be packaged in a rectangular, square, circular, oval or other shape and cut to the desired shape close to the time of application of the reuse engaging layer to the nasal support device.

In an alternative embodiment, a reusable nasal support device for animals can include one or more components as disclosed in the foregoing patents and patent applications with a "reusable engaging layer." According to this embodiment, the engaging layer can comprise an adhesive system which permits reapplication of a nasal support device to an animal after the nasal support device has previously been used on the same or a different animal without addition of a new engaging layer. One example of such a reusable adhesive which is suitable for this embodiment of the invention is a fibrous pressure-sensitive adhesive layer comprising an entangled web of pressure-sensitive adhesive fibers such as disclosed in U.S. Patent No. 5,957,126, the entire disclosure of which is incorporated herein by reference.

Thus, a support device as disclosed herein provides support for the nasal passages of an animal, particularly unsupported soft tissues. The devices can provide reduced resistance to air flow, including reduced turbulence, at rest and during exercise. In addition, the support devices can be used to treat or prevent respiratory ailments in adult or young animals.

Support of unsupported nasal tissues is particularly advantageous for animals such as horses because horses are obligate nose breathers. In preferred embodiments, a herein disclosed NSD may reduce the amount of bleeding which is associated with exercise-induced pulmonary hemorrhage (EIPH) in horses. Methods are known for determining the amount of pulmonary bleeding which occurs during EIPH. One such method includes performing pulmonary lavage post exertion and quantifying the number of red blood cells per microliter (i.e., RBC/µl) in the lavage fluid. In some embodiments, when a herein disclosed support device is worn during physical exertion by a horse susceptible to EIPH, pulmonary red blood cell counts can be reduced by at least 5%, preferably by at least 10-20%, in some embodiments by 30-40% and in some embodiments by at least 50-70% as compared to when the same horse is exercised without the NSD.

Without being limited to a particular theory, the inventors believe that support of the lateral vestibular wall, or portions thereof, over the nasal region of an animal decreases resistance to air flow and increases breathing efficiency. That is, when wearing an NSD, less energy is consumed by the animal during inspiration or expiration of air into the lungs. Resistance to air inflow/outflow is reduced by providing a cross sectional area of the nasal passages which is greater than the cross sectional area when the support device is not used. It is believed that a decrease in cross sectional area of the nasal passages requires an increase in intrapleural pressure (i.e., negative pressure) during inspiration to draw the same amount of air into the lungs. Airway resistance (R) is related to the pressure (P) across the nasal airway passage and flow (F) of air through the nasal passage by the equation: R = P/F.

The inventors believe that a high intrapleural negative pressure across pulmonary aveoli combined with high pulmonary blood pressure during exercise can cause rupture of pulmonary blood vessels which manifests as EIPH.

In some embodiments, when a herein disclosed support device is worn during exercise by a horse, nasal passage resistance can be reduced by at least about 5-10%, typically 20-30%, and in some animals, by greater than 40%. In addition, by reducing nasal passage resistance, less work is required during breathing resulting in reduced oxygen consumption and reduced CO₂ production for the same amount of exertion. Heart rate may also be reduced in a horse using an NSD when compared to the same horse performing at the same level of exertion without an NSD.

In another embodiment, the invention provides therapeutic devices for treating conditions amenable to the delivery of magnetic, electromagnetic, thermal (hot or cold), ultrasonic or other energy emitting modality. The device could also provide for delivery of a pharmaceutical agent. According to this embodiment, the surface layer and engaging layer components and constructions herein described for an NSD can be used. However, in contrast to previously described devices, in the present devices, the support layer is supplanted by, or supplemented with, a therapeutic layer. In addition, the configurational appearance can vary depending on the size or region of the body to which it is desired to deliver the therapeutic energy.

According to this embodiment, the therapeutic layer can include wafer shaped magnets, electromagnets, ultrasound transmitters, thermal emitters or other known therapeutic modality. For example, in one embodiment, the lift members can be supplanted by placement of thin magnets which deliver magnetic energy to a localized region of the body over which the device is placed. In general, preferred components of the therapeutic layer, such as magnets, are flexible to conform to the location of the body where the device will be applied, but lack significant elasticity or shape memory which would act to conform the shape of the body region to the engaging surface of the device. The adhesive surface can be continuous or intermittent on one side of the therapeutic layer. A surface layer may optionally be used.

In some embodiments, the device can include a surface layer, a therapeutic layer and an adhesive layer. The surface layer can be configured to include a border which extends sufficiently around the perimeter of the therapeutic layer. The adhesive layer can then be applied over the therapeutic layer and the perimeter border to provide adhesive engagement of the device to a selected location of the patient's body. As described for the support layer above, an adhesive can be used to adhere the therapeutic layer to the surface layer. Examples of appearances of suitable configurations can be understood by reference to FIGs. 3, 12-37 wherein the lift members would be replaced by the particular therapeutic delivery apparatus. For example, FIG. 42 illustrates one embodiment of a bottom plan view of therapeutic device **500.** As described for an NSD, the surface layer **501** can include an engaging layer **502** (transparent in this view) on the bottom surface **503** of surface layer **501** and therapeutic layer **504.** In this embodiment, therapeutic layer **504** can be a wafer magnet 505 or other apparatus for providing a particular therapeutic modality.

Many of the adhesive systems disclosed herein are advantageous for adhering to the body. In addition, by adjusting the moisture absorbing characteristics of the adhesives used, such as by addition of a hydrocolloid, such as carboxymethylcellulose, polyacrylamide, or similar composition, the ability to maintain position in the presence of sweat or other fluids will be increased. In addition to other adhesives, suitable adhesives include adhesives of a type used to maintain EKG patches on a human or animal body. In an alternative embodiment, the therapeutic deliver apparatus can be inserted into a pouch made from plastic, PTFE, fabric or other suitable material having a surface coated with an engaging layer as disclosed herein.

Examples of conditions which can be treated by placement of a herein disclosed therapeutic device at or near the location of the condition include: acute or chronic inflammation, non-union fractures, splints, muscle soreness, tendon injuries, etc.

Having now described the invention, it will be apparent to one of skill in the art that changes and modifications can be made to the invention without departing from the spirit or scope of the appended claims. All modifications and equivalents of the disclosed invention are intended to be included within the scope of the claims.

## Claims

1. A support device for supporting tissues overlying a first and second nasal passage, the support device comprising:
- an adhesive layer for securing the support device to the tissues;
- a support layer; and
- a surface layer, the surface layer configured to include:
- a first transverse dimension having a first transverse axis therethrough;
- a center longitudinal dimension having a center longitudinal axis which is orthogonal to said first transverse axis and bisects said first transverse axis;
- a first and second lateral longitudinal dimension on opposing sides of said center longitudinal dimension,
- said center longitudinal dimension greater than said first and second lateral longitudinal dimensions;
- said surface layer on opposing sides of said first transverse axis being mirror images of one another.

2. A support device according to claim 1 wherein said surface layer is bilateral symmetrical across both of said transverse and longitudinal axes.

3. A support device configured for supporting tissues overlying a first and second nasal passage of an animal, the support device comprising:
- a support layer; and
- a surface layer, the surface layer configured to include:
- a first axis which bisects said surface layer along a first dimension;
- a second axis which bisects said surface layer along a second dimension;
- said surface layer being bilaterally symmetrical across both of said first and second axes.

4. A support device according to claim 1 or claim 3
wherein said surface layer on opposing sides of said longitudinal axis are mirror images of one another.

5. A support device according to claim 1 or claim 3
wherein said center longitudinal dimension is less than said first transverse dimension.

6. A support device according to claim 3 wherein said support layer includes at least one lift member.

7. A support device for supporting tissues over a first and second nasal passage said device comprising;
- a first side piece for applying over said first nasal passage;
- a second side piece for applying over said second nasal passage; and
- a bridge piece having a first lateral region and a second lateral region for attaching said first lateral region to said first side piece and said second lateral region to said second side piece.

8. The support device according to claim 7 wherein said first and second lateral regions of said bridge piece attach to said first and second side pieces using velcro.

9. A therapeutic device comprising:
- a therapeutic layer; and
- an adhesive for maintaining said device in a selected location.

10. A support device for supporting tissues overlying a first and second nasal passage, the support device comprising:
- an engaging layer including an adhesive for engaging the support device to the tissues;
- a surface layer;
- a support layer positioned between the engaging layer and surface layer; and
- a carrier layer releasably mounted to the surface layer.

11. The support device according to claim 10 further comprising a release liner releasably attached to the adhesive of the engaging layer.

12. The support device according to claim 11 wherein the release liner comprises a first lateral piece, a second lateral piece, and an intermediate piece.

13. The support device according to claim 10 configured to include:
- a transverse axis having a transverse dimension;
- a center longitudinal axis having a center longitudinal dimension, the center longitudinal axis being orthogonal to the transverse axis and the center longitudinal axis bisects the transverse axis;
- a first and second lateral longitudinal dimension on opposing sides of the center longitudinal dimension;
- the center longitudinal dimension greater than the first and second lateral longitudinal dimensions;
- the surface layer on opposing sides of the transverse axis being mirror images of one another.

14. A support device according to claim 13 wherein the surface layer on opposing sides of the longitudinal axis are mirror images of one another.

15. A support device according to claim 1 or claim 10,
wherein the support layer includes at least two lift members.

16. A support device according to claim 1 or claim 10
wherein the support layer includes at least three lift members.

17. A support device according to claim 13 wherein the support device is bilaterally symmetrical across both of the transverse and longitudinal axes.

18. A support device according to claim 10 wherein the carrier layer includes a rostral extension.

19. A support device according to claim 18 wherein the rostral extension is an alignment guide.

20. A support device for supporting tissues overlying a first and second nasal passage, the support device comprising:
- an engaging layer including an adhesive for attaching the support device to the tissues;
- a surface layer;
- a support layer positioned between the engaging layer and surface layer; and
- a release liner releasably attached to the adhesive layer, the release liner comprising a first lateral piece, a second lateral piece, and an intermediate piece.

21. A support device according to claim 20 further comprising a carrier layer releasably mounted to the surface layer.

22. A support device according to claim 20 wherein the support layer includes at least two lift members.

23. A support device according to claim 16 or claim 20 having an engagement extension extending laterally beyond the lift members.

24. A support device according to claim 20 configured to include:
- a first side piece for engaging a first lateral vestibular wall overlying a first nasal passage, the first side piece having a rostral end, a caudal end and a first rostral-poll dimension;
- a second side piece for engaging a second lateral vestibular wall overlying a second nasal passage, the second side piece having a rostral end, a caudal end and a second rostral-poll dimension;
- a midline region including an intersection of the first and second side pieces, the midline region having a rostral end, a caudal end and a midline region rostral-poll dimension that is greater than either of the first rostral-poll dimension and the second rostral poll dimension.

25. A support device according to claim 20 wherein the support device is configured to fit the nose of a horse.

26. A support device according to claim 10 or claim 20
wherein the surface layer is a dark color.

27. A support device for supporting tissues overlying a first and second nasal passage, the support device comprising:
- an engaging layer including an adhesive for attaching the support device to the tissues;
- a surface layer;
- a support layer positioned between the engaging layer and surface layer;
wherein the engaging layer includes an engagement extension which extends laterally beyond the support layer.

28. A method for facilitating air flow through the nasal passages of an animal, the method comprising supporting a caudal apex region of a vestibular wall overlying the nasal passages of the animal.

29. A reusable nasal support device comprising:
- a support layer;
- a first engaging layer for adhesively securing the nasal support device to the nose of an animal; and
- a second engaging layer for adhesively securing the nasal support device to an animal.

30. The reusable nasal support device according to claim 29 wherein the second engaging layer is applied to the nasal support device after the first engaging layer has been adhesively secured and removed from the nose of an animal.

31. The reusable nasal support device according to claim 29 wherein the first engaging layer is a double-sided tape.

32. The reusable nasal support device according to claim 29 wherein the second engaging layer is a double-sided tape.

33. The reusable nasal support device according to claim 29 wherein the second engaging layer is a single-sided tape.

34. The reusable nasal support device according to claim 29 wherein the second engaging layer is a transfer adhesive tape.

35. The reusable nasal support device according to claim 29 further comprising three or more engaging layers.

36. A reusable nasal support device sized for an animal comprising:
- a support layer; and
- an engaging layer, the engaging layer comprising a fibrous pressure-sensitive adhesive layer that comprises an entangled web of pressure-sensitive adhesive fibers.
